# EUROPÄISCHE PATENTSCHRIFT

(11) **EP 0 288 070 B1**
(45) Veröffentlichungstag und Bekanntmachung des Hinweises auf die Patenterteilung: **10.02.1993**
(21) Anmeldenummer: 88106441.4
(22) Anmeldetag: 22.04.1988
(51) Int. Cl.: C04B 33/36, C04B 33/32, C04B 20/10, C04B 18/02, C04B 30/02

(54) **Tonhaltiges Material und Verfahren zur Herstellung desselben**
Clay-containing material and method for its preparation
Matériau argileux et son procédé de préparation

(30) Priorität: 24.04.1987 DE 3713735; 24.04.1987 DE 8705957 U; 09.06.1987 DE 3719153; 22.08.1987 DE 3743581; 03.09.1987 DE 3729371; 17.02.1988 DE 3804883
(43) Veröffentlichungstag der Anmeldung: 26.10.1988
(73) Patentinhaber: Broggini, Arturo, D-40476 Düsseldorf (DE)
(72) Erfinder: Broggini, Arturo, D-40476 Düsseldorf (DE)
(74) Vertreter: Fitzner, Ulrich, Dr.

(56) Entgegenhaltungen:
- EP-A- 0 206 989
- DE-A- 1 671 269
- DE-A- 1 910 735
- DE-A- 3 127 556
- GB-A- 2 093 014
- US-A- 3 510 394
- CHEMICAL ABSTRACTS, Band 106, Nr. 2, Januar 1987, Seite 230, Zusammenfassung Nr. 8717s, Columbus, Ohio, US; & JP-A-61 183 181 (NICHIAS CORP.) 15-08-1986

## Beschreibung

Die vorliegende Erfindung betrifft ein neues Verfahren zur Herstellung eines tonhaltigen Materials, das leicht und stabil ist, gegen Frost, Feuer und Witterung beständig ist und ein hohes Wärmedämmvermögen aufweist.

Tonhaltige Materialien werden heute in vielen Bereichen der Technik verwendet. Neben der Anwendung in der Keramik und Kunst zählt der Baustoffsektor zu den bevorzugten Einsatzgebieten. Aus Chemical Abstract 101:8717a. der DE-A-1671269, der GB-A-2093014 und der DE-A-1910735 sind die hierfür verwendbaren Zusammensetzungen bekannt. Hierbei werden jedoch regelmäßig neben den Fasern und den tonhaltigen Materialien noch Ausflockungs-, Binde- und Verkleisterungsmittel eingesetzt. Diese bisher bekannten tonhaltigen Materialien weisen jedoch einige Nachteile auf. So dauert der Herstellungsprozeß relativ lange (3 bis 6 Tage). Besonders viel Zeit nimmt das Trocknen von großen Teilen in Anspruch. Bei diesem Trocknungsvorgang kommt es zur Verdampfung von Wasser. Je mehr Wasser verdampft, desto größer ist die Gefahr von RiBbildung. Weiterer Nachteil herkömmlicher Materialien ist, daß sie vor dem Brand trocken sein müssen und bei schnellem Erhitzen und Kühlen Risse entstehen. Tonhaltige leichtere Materialien mit einem spezifischen Gewicht von weniger als 0,1 kg/cm³ sind nur in Verbindung mit Kunststoffen stabil. Nachteilig ist, daß diese Stoffe brennbar sind.

Die vorliegende Erfindung hat sich die Aufgabe gestellt, ein Verfahren zur Herstellung eines ton- und faserhaltigen Materials durch Beschichten von Mineral- oder Kohlefasern mit Ton oder mit Gemischen aus Bentonit und Titandioxid oder Ton und Quarzmehl oder Ton und Kalk, anschließendes Trocknen, Brennen bei 600 - 1600 °C und Abkühlen zur Verfügung zu stellen, das die vorerwähnten Nachteile nicht aufweist.

Diese Aufgabe wird dadurch gelöst, daß das Trocknen im Heißluftstrom bei mindestens 250 °C während einer Zeit von 1 bis 2 Stunden und das Brennen wenige Minuten bis etwa 1 Stunde durchgeführt wird und das Trocknen, Brennen und Abkühlen höchstens 6 bis 8 Stunden dauert.

Anstelle des tonhaltigen Materials können auch Tongemische oder lehmiger Ton verwendet werden. Die Tongemische können Bentonite, Titandioxid und ggf. andere mineralische Stoffe enthalten, je nach Tonmaterial und Anwendungsbereich.

Bei den Fasern handelt es sich vorzugsweise um Mineralfasern oder Kohlefasern. Glasfasern werden in Form von Wolle, Flocken, kleingeschnittenen Fasern und Matten eingesetzt.

Das tonhaltige Material kann dadurch hergestellt werden, daß Fasern mit Ton, Tongemischen aus Bentoniten und Titandioxid beschichtet werden. Das Beschichten geschieht in der Regel durch Eintauchen der Fasern in den flüssigen Ton oder ein flüssiges Tongemisch. Ebenso ist es aber auch möglich, im Spritzverfahren die Fasern zu beschichten. Besonders gute Ergebnisse werden bei der Herstellung im Vacuumverfahren erzielt.

Das so hergestellte tonhaltige Material kann zur Herstellung der verschiedensten gebrannten Tonerzeugnisse verwendet werden. Das erfindungsgemäße Verfahren zeichnet sich hierbei besonders dadurch aus, daß das Trocknen, Brennen und Abkühlen des Tonmaterials höchstens 8 Stunden dauert. Nach dem Stand der Technik waren allein für das Trocknen 1 bis 3 Tage erforderlich, der Brand dauerte mehrere Stunden, teilweise 8 bis 20 Stunden.

Mit dem erfindungsgemäßen Verfahren konnte nunmehr eine überraschend große Abkürzung der gesamten Herstellungszeit erreicht werden. Für Trocknen, Brennen und Abkühlen sind nunmehr nicht mehr als je 6 Stunde erforderlich. Das Trocknen im Heißluftstrom bei 250 bis 300°C kann innerhalb weniger Minuten sogar abgeschlossen werden. Die Wasserverdampfung hat keine Verformung und Rißbildung zur Folge. Das Wasser verdampft zwischen den Fasern, hat jedoch nicht genügend Kraft die Fasern zu verschieben. Die Folge ist, daß bei der Trocknung des erfindungsgemäßen tonhaltigen Materials kaum Schrumpfungen auftreten. Dadurch wird gleichzeitig die Gefahr der Rißbildung ausgeschlossen.

Je nach eingesetztem Material kann das tonhaltige Material bei Temperaturen zwischen 600 und 1600°C gebrannt werden. Sofern in dem Material mineralische Glasfasern enthalten sind, erfolgt das Brennen normalerweise bei 900°C. Mineralfaserhaltige Materialien werden normalerweise bei 900 bis 1200°C gebrannt. Mineralische Glasfasern weisen einen Schmelzpunkt von etwa 650°C auf. D.h., daß der Tonmantel bei gleicher Temperatur eine Kruste bildet, die die flüssigwerdende mineralische Glasfaser umhüllt. Bei 850-900 °C erfolgt die Sinterung beider Stoffe. Demnach liegen die Temperaturen in der Regel bei 800-1000°C. Insbesondere bei Verwendung von mineralischen Fasern mit hohem Schmelzpunkt erhöhen sich Sintertemperaturen bis zu 1600°C.

Die gebrannten Materialien zeichnen sich durch ein besonders geringes Gewicht aus. Ebenso sind sie gegen Witterung und chemische Einflüsse beständig und daher auch sehr umweltfreundliche Materialien. Ferner ist eine ausgezeichnete Belastbarkeit beobachtet worden. Und zwar halten die erfindungsgemäßen Materialien besonderen Belastungen stand, die zur Faserrichtung ausgeübt werden.

Eine gegenüber herkömmlichen Tonmaterialien völlig neue Eigenschaft ist, daß die erfindungsgemäß hergestellten Materialien sich zusammenschweißen lassen durch Flammensintern. Ebenso sind sie jedoch durch übliche Bindemittel verklebbar. Ebenso wie übliche Tonmaterialien können auch die erfindungsgemäßen Materialien weiter glasiert, mit Kunststoffen, Reaktionsharze, Imprägnierungsmitteln beschichtet oder getränkt werden.

Die erfindungsgemäß hergestellten Materialien können auf vielfältigen Gebieten eingesetzt werden. So können sie als Bausteine, Bauplatten oder Isoliermaterialien dienen. Weitere Anwendungsgebiete sind die Autoindustrie, die Baustoffindustrie und Filteranlagen. Sehr gut geeignet sind die Materialien für Schalungen. Aufgrund des geringen spezifischen Gewichtes kann man extrem leichte Baustoffe erhalten. Weiterhin eignen sich die Materialien als Holzersatzstoffe, Fassadenplatten, Möbel und Einrichtungselemente, als Trägermaterial für Festbettreaktoren, für die Immobilisierung von Mikroorganismen oder Enzymen, für Biotope und Riffbau, Ofenbau, Feuerschutzelemente, Orthopädie und Herstellung künstlicher Knochen, Gelenke usw. Die Materialien lassen sich gut schneiden und bohren. Es können ebenso wie beim Holz stabile Schraub- oder Klebverbindungen hergestellt werden. Der Vorteil gegenüber Holz liegt in der Witterungsbeständigkeit, Feuerbeständigkeit, dem geringeren Gewicht und der Beständigkeit gegen chemische Einflüsse. Aufgrund dieser guten Eigenschaften sind auch Anwendungen in der Raumfahrt möglich.

## Patentansprüche

1. Verfahren zur Herstellung eines ton- und faserhaltigen Materials durch Beschichten von Mineral- oder Kohlefasern mit Ton oder Gemischen aus Bentonit und Titandioxid oder Ton und Quarzmehl oder Ton und Kalk, anschließendes Trocknen, Brennen bei 600 1600°C und Abkühlen,
**dadurch gekennzeichnet,** daß das Trocknen im Heißluftstrom bei mindestens 250°C während einer Zeit von ein bis Zwei Stunden und das Trocknen, Brennen und Abkühlen höchstens acht Stunden dauert.

2. Verfahren nach Anspruch 1,
**dadurch gekennzeichnet,** daß das Trocknen, Brennen und Abkühlen des Tonmaterials insgesamt drei bis sechs Stunden dauert.

3. Verfahren nach einem der Ansprüche 1 oder 2,
**dadurch gekennzeichnet,** daß das Brennen und Abkühlen innerhalb von einer bis sechs Stunden durchgeführt wird.

4. Verfahren nach einem der Ansprüche 1 bis 3,
**dadurch gekennzeichnet,** daß das Trocknen im Heißluftstrom bei 250 - 300°C durchgeführt wird.

5. Verfahren nach einem der Ansprüche 1 bis 4,
**dadurch gekennzeichnet,** daß das Brennen bei 950°C in wenigen Minuten bis etwa einer Stunde durchgegeführt wird.

## Claims

1. A process for the manufacture of a material containing clay and fibres by coating mineral or carbon fibres with clay or mixtures of bentonite and titanium dioxide or clay and quartz flour or clay and lime, subsequent drying, calcining at 600 - 1600°C and cooling,
**characterized in that** drying in the hot-air current at at least 250°C takes place for a period of one to two hours and drying, calcining and cooling takes 8 hours at the most.

2. The process according to claim 1,
**characterized in that** drying, calcining and cooling of the clay material takes three to six hours altogether.

3. The process according to any of claims 1 or 2,
**characterized in that** calcining and cooling is accomplished within one to six hours.

4. The process according to any of claims 1 to 3,
**characterized in that** drying is performed in the hot-air current at 250 - 300°C.

5. The process according to any of claims 1 to 4,
**characterized in that** calcining at 950°C is performed within a few minutes up to approximately one hour.

## Revendications

1. Procédé pour la fabrication d'un materiau contenant de l'argile et des fibres obtenu par le revêtement de fibres minérales ou de fibres carboniques avec de l'argile,ou par un mélange de bentonite et des dioxydes de titane,ou de l'argile et de la farine de quartz, ou encore de l'argile et de la chaux , puis par séchage et cuisson du mélange à une témpérature entre 600° C et 1600° C et le laisser réfrodir

2. Procédé selon la revendication 1 caractérisé par le fait que le séchage, la cuisson, et le réfroidissement du materiau argileu durent entre trois et six heures

3. Procédé selon la revendication 1 ou 2 caractérisé par le fait que la cuisson et le réfroidissement sont exécutés entre une et six heures.

4. Procédé selon les revendications de 1 à 3 caractérisé par le fait que le séchage au moyen d'un courant d'air chaud soit exécuté à une témpérature entre 250° C et 300° C .

5. Procédé selon les revendications de 1 à 4 caractérisé par le fait que la cuisson à une témpérature de 950° c soit exécutée entre quelques minutes et environ une heure.
